Europäisches Patentamt

European Patent Office

Office européen des brevets

(19)

(11) **EP 1 281 964 A2**

(12) **EUROPÄISCHE PATENTANMELDUNG**

(43) Veröffentlichungstag:
**05.02.2003 Patentblatt 2003/06**

(51) Int Cl.⁷: **G01N 33/542**, G01N 33/58

(21) Anmeldenummer: **02017252.4**

(22) Anmeldetag: **31.07.2002**

(84) Benannte Vertragsstaaten:
**AT BE BG CH CY CZ DE DK EE ES FI FR GB GR
IE IT LI LU MC NL PT SE SK TR**
Benannte Erstreckungsstaaten:
**AL LT LV MK RO SI**

(30) Priorität: **01.08.2001 DE 10137530**

(71) Anmelder: **Chromeon GmbH
93053 Regensburg (DE)**

(72) Erfinder:
• **Klimant, Ingo
93098 Mintraching (DE)**
• **Kürner, Jens
93053 Regensburg (DE)**

(74) Vertreter: **Weiss, Wolfgang, Dipl.-Chem. Dr. et al
Weickmann & Weickmann
Patentanwälte
Kopernikusstrasse 9
81679 München (DE)**

(54) **Anordnung und Verfahren zur Mehrfach-Fluoreszenzmessung**

(57) In Nanopartikel werden gemeinsam ein phosphoreszierender Donorfarbstoff und mehrere fluoreszierende Akzeptorfarbstoffe immobilisiert. Diese Nanopartikel dienen als Multiplex-Marker für eine Vielzahl von Analyten, die sowohl nach den Absorptionsspektren der Akzeptorfarbstoffe als auch nach den Lumineszenz-Abklingzeiten der jeweiligen Farbstoffe ausgewertet werden können.

CY604. (A) Absorptionsspektren in Phosphatpufferlösung (Farbstoffkonzentration von oben nach unten: 51.42, 25.71, 10.28, 5.14, 0 µmol/L). (B) Emissionsspektren in Phosphatpufferlösung (Farbstoffkonzentration von oben nach unten: 51.42, 25.71, 10.28, 5.14, 0 µmol/L). (C) Multifrequenzspektren in Phosphatpufferlösung (Farbstoffkonzentration - Modulation von oben nach unten / Phasenwinkel von unten nach oben: 51.42, 25.71, 10.28, 5.14, 0 µmol/L).

FIG. 7

EP 1 281 964 A2

**Beschreibung**

Die Erfindung betrifft eine Anordnung und ein Verfahren zur Mehrfach-Fluoreszenzmessung durch eine Mehrzahl gemeinsam immobilisierter Fluoreszenzmarker, insbesondere in Mikro- oder Nanopartikeln. Die Farbstoffe haben überlappende Absorptionsspektren, sodass bei Erregung eines Farbstoffs eine Energieübertragung zum benachbarten Farbstoff stattfindet. Hierdurch vervielfacht sich die messbare Lichtausbeute, was diese Partikel für einen hochempfindlichen Nachweis von Substanzen, insbesondere Biomolekülen geeignet macht, an die sie gebunden sind, etwa beim Nachweis von RNA oder DNA, in der Durchflusszytometrie, mikroskopischen Analysetechniken, wie Licht oder Fluoreszenzmikroskopie, oder auch konfokaler 3D-Mikroskopie zur Diagnostik, Analytik, Medizin, Immunoassays.

Die Farbstoffe werden so ausgewählt, dass sie bei hoher Lichtausbeute eine möglichst große Stokes-Verschiebung erreichen, um Anregung und Signal der Farbstoffe ohne große Lichtverluste separieren zu können.

Weitere wichtige Eigenschaften der Farbstoffe sind eine langfristige Fotostabilität. Die Lumineszenzeigenschaften sollen von der Probe nicht beeinflusst werden. Es müssen reaktive Gruppen vorhanden sein, um an das nachzuweisende Molekül selektiv zu koppeln. Die Farbstoffe sollen wasserlöslich und ungiftig sein.

Obwohl eine Vielzahl verschiedener Fluoreszenzfarbstoffe als Marker bekannt sind, zeigte es sich, dass nur wenige dieser Farbstoffe alle der oben genannten Kriterien erfüllen.

Problematisch ist insbesondere eine nicht ausreichende Helligkeit der Messsignale, vor allem bei solchen Proben, die eine hohe Untergrund-Fluoreszenz besitzen.

Ferner besteht ein großer Bedarf an unterschiedlichen Farbstoffen mit klar unterscheidbaren Merkmalen (Multiplex-Farbstoffe), um eine große Anzahl unterschiedlich markierter biologischer Proben, wie z.B. DNA-Fragmente oder Proteine, voneinander unterscheiden zu können.

Um die Helligkeit von Lumineszenzassays zu erhöhen und die inhärente Untergrundfluoreszenz der Probe zu eliminieren, kann man etwa die Fluoreszenzfarbstoffe in die einleitend erwähnten polymeren Matrizes, etwa Mikro- oder Nanopartikel, einbauen, was nicht nur die Quantenausbeute erhöht, sondern die Farbstoffe gleichzeitig vor unerwünschten Einflüssen seitens der Matrix abschirmt, insbesondere Löschung. Im Übrigen ermöglicht der Einbau einer Vielzahl unterschiedlicher Farbstoffmoleküle in einen einzelnen Partikel eine deutliche Erhöhung der Signalintensität in einem Lumineszenzassay.

Um die Helligkeit zu verbessern und die Untergrundfluoreszenz der Probe zu beseitigen, kann man ferner langwellig emittierende Lumineszenzfarbstoffe verwenden. Diese ermöglichen die selektive Detektion von Luminszenzsignalen in natürlichen Proben, wie etwa Körperflüssigkeit, da nur wenige natürliche Verbindungen rotes Licht emittieren.

Eine weitere Möglichkeit, die Helligkeit zu verbessern und die Untergrundfluoreszenz zu eliminieren, besteht in der Verwendung von phosphoreszierenden Farbstoffen. Da die Eigenfluoreszenz in den meisten Proben in der Regel nach wenigen Nanosekunden vollständig abgeklungen ist, ermöglicht die Verwendung von phosphoreszierendem Farbstoff aufgrund deren langer Abklingzeit eine zeitlich aufgelöste Messung und somit eine Untergrundfluoreszenzfreie Detektion der Fluoreszenzsignale.

Typische häufig eingesetzte Fluoreszenzfarbstoffe sind dabei die Chelate der Seltenerdenmetalle (Eu 3+, Tb 3+).

Die oben erwähnten sogenannten Multiplex-Farbstoffe oder Multiplex-Marker können herkömmlich wie folgt hergestellt werden:

1. Man verwendet eine Serie von Farbstoffen mit unterschiedlichen spektralen Eigenschaften im Hinblick auf Absorption und Emission.

2. Man verwendet Mikropartikel mit jeweils mehreren eingebauten Farbstoffen, die gleiche Absorptions- aber unterschiedliche Emissionseigenschaften haben.

3. Man verwendet Mikropartikel mit zwei eingebauten Farbstoffen, die sich spektral voneinander unterscheiden, etwa Identifizierung über radiometrische Messung zweier Lumineszenzintensitäten; und

4. man verwendet eine Serie von Farbstoffen mit unterschiedlichem Abklingverhalten, aber identischen spektralen Eigenschaften.

Alle diese herkömmlichen Konzepte unterliegen jedoch Einschränkungen: Es lässt sich nur eine begrenzte Anzahl an unterschiedlichen Markern herstellen, maximal 6 bis 10. Ferner erfordern die obigen Konzepte 1, 2 und 4 für jeden einzelnen Marker einen individuellen Fluoreszenzfarbstoff, während das Konzept 3 immerhin nur zwei individuelle Farbstoffe benötigt, um eine ganze Palette von Markern bereitzustellen.

Für die Multianalyt-Detektion, die insbesondere in der DNA und Immunanalytik immer wichtiger wird, benötigt man aber eine wesentlich größere Anzahl an eindeutig unterscheidbaren Markierungsfarbstoffen, insbesondere zur Zellsortierung, Durchflusszytometrie, zur Immun- und DNA-Chips und zur Fluoreszenzmikroskopie.

Aus der US-A-5,326,692 ist es bekannt, eine Kaskade spektral überlappender Fluoreszenzfarbstoffe in Nanopartikel zu immobilisieren.

Aufgabe der Erfindung ist es daher, eine Anordnung und ein Verfahren zur fluorometrischen Messung einer Probe anzugeben, die bzw. das eine problemlosere Mehrfach-Messung einer Vielzahl von Lumineszenzsignalen gestattet.

Zur Lösung der Aufgabe wird vorgeschlagen, einen lumineszierenden Donorfarbstoff und mehrere mit dem Donorfarbstoff immobilisierte, durch Energietransfer von dem Donorfarbstoff lumineszierende Akzeptorfarbstoffe vorzusehen, wobei der Donorfarbstoff als Phosphoreszenzfarbstoff ausgeführt ist und die jeweiligen Akzeptorfarbstoffe als Fluoreszenzfarbstoffe ausgeführt sind.

Im Gegensatz zur US-A-5,326,692 wird als Donorfarbstoff kein Fluoreszenzfarbstoff, sondern ein Phosphoreszenzfarbstoff verwendet, wohingegen die Akzeptorfarbstoffe aus üblichen Fluorophoren ausgewählt werden können. Die breite Emissionsbande von Phosphoreszenzfarbstoffen als Donor erlaubt es, diesen Donor mit einer Reihe unterschiedlicher Akzeptorfarbstoffe zu kombinieren, um unterschiedliche spektrale Eigenschaften zu erhalten.

Jede Donor/Akzeptorpaarung reagiert in vorbestimmter Weise auf einen bestimmten Analyten.

Trotz Verwendung mehrerer unterschiedlicher Akzeptorfarbstoffe genügt ein einziger Donorfarbstoff, bevorzugt ein hochlumineszierender Ru(II)-Polypyridylkomplex, der mit diesen unterschiedlichen Akzeptorfarbstoffen kombiniert werden kann. Vorzugsweise wird ein Donorfarbstoff mit einer langen Lumineszenzabklingzeit von z.B. 100 ns bis 100 μs, insbesondere vorzugweise eine lange Lumineszenzabklingzeit hinsichtlich der durch den Donorfarbstoff stimulierten Emission, zB. $\geq 50$ ns, vorzugsweise 50 ns bis 10 μs.

Die mehreren unterschiedlichen Akzeptorfarbstoffe, die gemeinsam mit dem Donorfarbstoff immobilisiert sind, können sich in den Emissionsspektren voneinander unterscheiden. Auf diese Weise erhält man eine eindimensionale Serie an Fluoreszenzmarkern mit identischem Absorptionsverhalten aber spektral klar differenzierbaren Emissionseigenschaften. Variiert man jetzt noch die Konzentration des Akzeptorfarbstoffs, so dass ein Akzeptorfarbstoff jeweils separat in mehreren unterschiedlichen Konzentrationen mit dem Donorfarbstoff immobilisiert ist, verändert man hierdurch auch das zeitliche Abklingverhalten des Donorfarbstoffs und gleichzeitig auch das zeitliche Abklingverhalten der stimulierten Fluoreszenz des jeweiligen Akzeptorfarbstoffs.

Damit ist es möglich, neben den spektralen Eigenschaften des Akzeptorfarbstoffs auch die Lumineszenzabklingzeiten der Anordnung, d.h. des Donorfarbstoffs und/oder des jeweiligen Akzeptorfarbstoffs als Parameter zur Identifizierung des Analyten heranzuziehen. Man erhält somit ein zweidimensionales Feld an Lumineszenzmarkern, wobei die erste Dimension durch die spektralen Emissionseigenschaften des Akzeptors definiert ist und die zweite Dimension durch das zeitliche Abklingverhalten des Donors und/oder Akzeptors abhängig von der jeweiligen Konzentration.

Bei den Akzeptorfarbstoffen handelt es sich bevorzugt um Carbocyaninfarbstoffe, die in zahlreichen Varianten im Handel erhältlich sind. Diese Carbocyanine zeigen nämlich keine Eigenabsorption bei der Erregungswellenlänge des Rutheniumkomplexes des Donors, nämlich bei 488 nm. Es können bis zu zehn verschiedene Akzeptorfarbstoffe gleichzeitig mit einem gemeinsamen Donorfarbstoff immobilisiert werden. 488 nm. Es können bis zu zehn verschiedene Akzeptorfarbstoffe gleichzeitig mit einem gemeinsamen Donorfarbstoff immobilisiert werden.

Bevorzugt sind der Donorfarbstoff und die Akzeptorfarbstoffe gemeinsam mit einer Kunststoffmatrix immobilisiert, wobei der Donorfarbstoff eine Konzentration von 1 bis 15 Gew.-%, bevorzugt ca. 10 Gew.-% einnehmen kann, ohne dass die Quantenausbeute signifikant herabgesetzt wird. Die fehlende Überlappung von Absorption und Emission des Donormoleküls verhindert eine Selbstlöschung. Dies führt zu einer extrem hohen Helligkeit der Lumineszenzsignale.

Bevorzugt sind der Donorfarbstoff und die Akzeptorfarbstoffe in Mikrooder Nanopartikel eingebettet, bevorzugt einer Größe von ca. $\leq 50$ μm, bestehend aus polymerisierten Monomeren, z.B. Acrylaten, Styrolen, ungesättigten Chloriden, Estern, Acetaten, Amiden, Alkoholen etc., insbesondere etwa Polymethyl-methacrylat-Partikeln oder solchen aus Polystyrol. Die Partikel können auch beschichtet sein, um deren Oberflächenstruktur zu modifizieren.

Diese Mikro- oder Nanopartikel können durch Fällung einer Lösung von Poylnitril in Dimethylformamid (DMF) hergestellt werden, wobei dann gleichzeitig die Farbstoffe mit in die Partikel eingebettet werden.

Die Erfindung betrifft ferner ein Verfahren zur gleichzeitigen fluorometrischen Messung mehrerer Analyten, insbesondere mit einer Anordnung der obigen Art mit den Schritten:

- Anregen des Donorfarbstoffs und
- Messen und Auswerten der spektral unterschiedlichen Fluoreszenzantworten der Akzeptorfarbstoffe, und
- Messen und Auswerten der durch die Fluoreszenzsignale der Akzeptorfarbstoffe in Wechselwirkung mit dem Donorfarbstoff beeinflussten Lumineszenzabklingzeiten der Anordnung.

Die Fluoreszenzantworten bzw. Fluoreszenzabklingzeiten können mit dem Vorhandensein bzw. mit der Konzentration der zu bestimmenden Analyten nach grundsätzlich bekannten Methoden korreliert werden.

Man erhält somit ein zweidimensionales Feld an Lumineszenzmarkern, definiert durch das spektrale Verhalten der Akzeptorfarbstoffe und das zeitliche Verhalten der Anordnung.

Die Messung und Auswertung der Fluoreszenzantworten der Akzeptorfarbstoffe oder/und der durch die Fluoreszenzsignale der Akzeptorfarbstoffe in Wechselwirkung mit dem Donorfarbstoff beeinflussten Lumineszenzabklingzeiten erfolgt vorzugsweise zeitlich aufgelöst, um das Untergrundsignal zu reduzieren. Besonders bevorzugt wird ein zeitliches Messfenster eingestellt, so dass die Messung erst nach weitgehendem Abklingen des Untergrundsignals mit kurzer Abklingdauer von z.B. <50 ns beginnt.

Mit der erfindungsgemäßen Messanordnung erhält man folgende Eigenschaften:

1. Die durch Energietransfer induzierte Fluoreszenz des Akzeptors klingt langsam ab, nämlich im Bereich von Mikrosekunden, und trägt damit die zeitlichen Abklingeigenschaften der Phosphoreszenz. Mit zeitaufgelösten Methoden der Phosphoreszenzdetektion ist damit ein untergrundfreies Messen möglich.

2. Es lässt sich ein 2D-Feld an Fluoreszenzmarkern realisieren. Mit sieben verschiedenen Farbstoffen (ein Donor, sechs Akzeptoren) und zehn individuell unterscheidbaren Abklingzeiten können somit 60 unterscheidbare Marker realisiert werden.

3. Die Emissionen aller Marker können mit einem blauen Argonlonenlaser angeregt werden. Aufgrund der besonders effizienten Lichtabsorption des Rutheniumkomplexes als Donor auch bei einer Wellenlänge von 404 nm, lassen sich auch blaue Laserdioden als Lichtquelle benutzen.

4. Die Stokes-Verschiebung aller Marker ist außergewöhnlich groß. Bei Verwendung der blauen Laserdiode als Lichtquelle liegt die Stokes-Verschiebung zwischen 190 nm und 360 nm. Dies wäre nach dem US-Patent 5,326,692 nur mit einer außerordentlich langen Kaskade vieler Farbstoffe erreichbar, wobei ein großer Helligkeitsverlust in Kauf genommen werden müsste, da jede Kaskadenstufe einen zusätzlichen Signalverlust hervorruft. Diese großen Stokes-Verschiebungen sind durch die spektralen Eigenschaften des Donors bedingt.

5. Der Einbau der phosphoreszierenden Donormoleküle in eine Polymermatrix mit geringer Sauerstoff-Permeabilität verhindert eine Löschung der Phosphoreszenz und verbessert die Signalintensitäten.

6. Durch die Verwendung von Polyacrylnitril-Copolymerisat als Matrix lassen sich phosphoreszierende Nanopartikel mit reaktiven Oberflächen zum Koppeln von Biomolekülen herstellen. Die Beladungsdichte der Oberflächen mit reaktiven Gruppen kann über die Eigenschaften des Copolymers eingestellt werden.

7. Verwenden lassen sich unterschiedliche Partikel, z.B. auch Latexpartikel, die nachträglich angefärbt werden, wobei der Einbau der Farbstoffe während der Emulsions-Polymerisation erfolgt. Nachfolgend werden Ausführungsbeispiele der Erfindung beschrieben.

1. Herstellung der Ausgangslösungen

Zur Herstellung der Farbstofflösungen A, B1 bis B4 und C1a bis C1e, C2a bis C2e, C3a bis C3e und C4a bis C4e wurden die in den Tabellen 1 bis 6 aufgelisteten Ansätze hergestellt. Hierbei gelten folgende Abkürzungen:

RU              $Ru(dph-phen)_3(TMS)_2$ als Donorfarbstoff
PAN-COOH        Poly-(acrylnitril-co-acrylsäure) (5 Gew.-% Acrylsäure) als Matrixmaterial
CY582           3,3'-Diethyloxadicarbocyanin-Jodid (99 %) als Akzeptor
CY604           1,1'-Diethyl-2,2'-carbocyaninchlorid als Akzeptor
CY655           3,3'-Diethylthiadicarbocyanin-Jodid (98 %) als Akzeptor
CY703           1,1'-Diethyl-4,4'-carbocyanin-Jodid (96 %) als Akzeptor.

Die Polyacrylnitrilmatrix und die Ruthenium- und Carbocyaninfarbstoffe sind in N,N-Dimethylformamid (DMF) als Lösungsmittel vollständig lösbar.

Tabelle 1

| Herstellung der Ruthenium-Donorlösung A | |
|---|---|
| Lösung | A |
| M (RU) [g/mol] | 1404,80 |
| m (RU) [mg] | 7,024 |
| n (RU) [μmol] | 5,0 |
| m (PAN-COOH) [g] | 1,0 |
| V (DMF) [ml] | 100 |

Tabelle 2

| Herstellung der Carbocyanin-Akzeptorlösungen B1-B4 | | | | |
|---|---|---|---|---|
| Lösung | B1 | B2 | B3 | B4 |
| Farbstoff | CY582 | CY604 | CY655 | CY703 |
| M (Farbstoff) [g/mol] | 486,36 | 388,94 | 518,48 | 480,39 |
| m (Farbstoff) [mg] | 6,0 | 5,0 | 5,0 | 20,0 |
| n (Farbstoff) [μmol] | 12,3 | 12,9 | 9,6 | 41,6 |
| V (DMF) [mL] | 60 | 50 | 50 | 75 |

Tabelle 3

| Herstellung der Energietransferlösungen C1a-C1e | | | | | |
|---|---|---|---|---|---|
| Lösung | C1a | C1b | C1c | C1d | C1e |
| V (A) [mL] | 5,0 | 5,0 | 5,0 | 5,0 | 5,0 |
| V (B1) [mL] | 0 | 0,5 | 1,0 | 2,5 | 5,0 |
| V (DMF) [mL] | 5,0 | 4,5 | 4,0 | 2,5 | 0 |

Tabelle 4

| Herstellung der Energietransferlösungen C2a-C2e | | | | | |
|---|---|---|---|---|---|
| Lösung | C2a | C2b | C2c | C2d | C2e |
| V (A) [mL] | 5,0 | 5,0 | 5,0 | 5,0 | 5,0 |
| V (B2) [mL] | 0 | 0,5 | 1,0 | 2,5 | 5,0 |
| V (DMF) [mL] | 5,0 | 4,5 | 4,0 | 2,5 | 0 |

Tabelle 5

| Herstellung der Energietransferlösungen C3a-C3e | | | | | |
|---|---|---|---|---|---|
| Lösung | C3a | C3b | C3c | C3d | C3e |
| V (A) [mL] | 5,0 | 5,0 | 5,0 | 5,0 | 5,0 |
| V (B3) [mL] | O | 0,5 | 1,0 | 2,5 | 5,0 |
| V (DMF) [mL] | 5,0 | 4,5 | 4,0 | 2,5 | 0 |

Tabelle 6

| Herstellung der Energietransferlösungen C4a-C4e | | | | | |
|---|---|---|---|---|---|
| Lösung | C4a | C4b | C4c | C4d | C4e |
| V (A) [mL] | 5,0 | 5,0 | 5,0 | 5,0 | 5,0 |
| V (B4) [mL] | 0 | 0,5 | 1,0 | 2,5 | 5,0 |
| V (DMF) [mL] | 5,0 | 4,5 | 4,0 | 2,5 | 0 |

2. Herstellung phosphoreszierender Nanopartikel

Zur Herstellung der Partikel wurden 1 g des Polyacrylnitril/Polyacrylsäure-Copolymers in 200 ml trockenem Dimethylformamid gelöst. 20 mg des Donorfarbstoffs mit unterschiedlichen Anteilen des entsprechenden Akzeptorfarbstoffs wurden darin gelöst. Hierzu wurden 400 ml destilliertes Wasser zugetropft, um das Polyacrylnitril (PAN) als Nanopartikel auszufällen. Man erhält dann eine klare phosphoreszierende Lösung. Nach 1 Stunde Wartezeit werden eine normale HCl-Lösung zugegeben, um die gefärbten Partikel agreggieren zu lassen. Danach wird die entstandene Suspension zentrifugiert und mit destilliertem Wasser gewaschen. Dann wird der Niederschlag in einem Phosphatpuffer mit pH 7,0 aufgenommen und im Ultraschall redispergiert. Nach Erwärmung auf 70 °C über 15 Min. waren die Suspensionen klar und über mehrere Wochen stabil. Sie wurden lichtgeschützt bei 10 °C aufbewahrt.

3. Meßaufbau

Es werden ein Donorfarbstoff und mehrere Akzeptorfarbstoffe innerhalb desselben Polyacrylnitril-Nanopartikel eingebettet, sodass ein Energietransfer zwischen den Farbstoffen ermöglicht wird. Die zusätzlichen reaktiven Carboxylgruppen an der Oberfläche des Partikels erleichtern das Ankoppeln der Nanopartikel über kovalente Bindungen an Proteine und andere Biomoleküle.

Korrigierte Fluoreszenz-Emissionsspektren zum Berechnen der Quantenausbeute wurden unter Verwendung der folgenden Gleichung (1) erhalten. Hierbei ist $\phi$ die Quantenausbeute, $A(\lambda)$ ist die Absorption pro Zentimeter der Lösung bei der Erregungswellenlänge $\lambda$, $I(\lambda)$ ist die relative Intensität des Erregungslichts bei der Wellenlänge $\lambda$, n ist der durchschnittliche Berechnungsindex der Lösung für die Lumineszenz und D ist das Flächenintegral unter dem korrigierten Emissionsspektrum. Die Indexe x und R betreffen die unbekannten bzw. die Referenz (Ruthenium(II)tris (2,2-bipyridyl)chloridhexahydrat)-Lösungen.

$$\Phi_x = \Phi_R \cdot \left( \frac{A_R(\lambda_R)}{A_x(\lambda_x)} \right) \cdot \left( \frac{I(\lambda_R)}{I(\lambda_x)} \right) \cdot \left( \frac{n_x^2}{n_R^2} \right) \cdot \left( \frac{D_x}{D_R} \right)$$

Da während der Messungen der Quantenausbeute die Spannung des Detektors konstant gehalten wurde, und da alle Lösungen wässrige Lösungen waren, ließen sich folgende Vereinfachungen durchführen:

$$I(\lambda_R) \approx I(\lambda_x) \text{ und } n_x \approx n_R.$$

Mehrfrequenz-Phasenmessungen (1 kHz bis 1 MHz) wurden an einem ISS K2-Mehrfrequenz-Phasenfluorometer durchgeführt. Die Abklingzeitmessungen erfolgten in der Frequenzdomäne. Durchschnittliche Abklingzeiten $T$ wurden aus den Phasenwinkeln $\theta$ berechnet, die durch eine Einzelfrequenzmessung erhalten wurden, gemäß folgender Gleichung (2):

$$\tau = \frac{\tan \theta}{2\pi \cdot f}$$

Als Lichtquelle verwendet wurde eine helle Blaulicht-emittierende Diode (LED) ($\lambda_{max}$ = 470 nm, NSPB 500, Nichia, Nürnberg, Deutschland), ausgestattet mit einem Blauglasfilter (BG 12, Schott, Mainz, Deutschland). Als Erfassungseinheit verwendet wurde eine kompakte rot-empfindliche Fotomultiplier-Röhre (H5701-02, Hamamatsu, Herrsching, Deutschland), ausgestattet mit einem Sperrfilter (OG 570, Schott). Das Erregungslicht der LED wurde bei einer Frequenz f von 45 kHz Sinuswellen-moduliert unter Verwendung eines doppelphasigen Lock-In-Verstärkers (DSP 830, Stanford Research, Sunnyvale/California, USA).

Der Verstärker wurde auch zum Messen der Phasenverschiebung der emittierten Lumineszenz benutzt. Ein gegabeltes Faserbündel mit Glasfasern (NA 0,46, d = 2mm) wurde mit einer thermostatisierten Zelle (T = 25 °C) verbunden, wobei die Spitze des Faserbündels in die umgerührte Meßlösung eingetaucht wurde.

4. Wahl der Matrix und der Farbstoffe

Das Poly(acrylonitril-co-acrylsäure)copolymer ist eine ausgezeichnete Matrix, da sie eine geringe Gasdurchlässigkeit hat und daher eingelagerte Lumineszenzfarbstoffe vor Gas wie etwa Sauerstoff schützt, was zu vernachlässigbaren Quenching-Effekten führt. Ferner versehen die Carboxylgruppen das Copolymer mit reaktiven Gruppen zur kovalenten Bindung an andere Moleküle.

Polyacrylnitril-Derivate bilden eine brauchbare Matrix zum Einbetten organischer phosphoreszierender Farbstoffe, da sie eine geringe Durchlässigkeit für Gase und gelöste ionische und neutrale chemische Verbindungen haben. Daher werden die Farbstoffe effizient gegen Lumineszenzquenching abgeschirmt, beispielsweise aufgrund von molekularem Sauerstoff, und daher zeigen sie konstante Abklingzeiten und Quantenausbeuten in Proben variabler und unbekannter Zusammensetzungen. Zusätzlich sind auch viele lipophile Farbstoffe in diesen Materialien gut löslich und werden nicht in die Probe ausgewaschen.

Die Nanopartikel haben ein sehr hohes Oberflächenvolumenverhältnis. Polyacrylnitril mit einem Polyacrylsäuregehalt von 5 % erwies sich als eine besonders brauchbare Einbettungsmatrix. Suspensionen solcher phosphoreszierender Nanopartikel sind durch Sauerstoff praktisch nicht quenchbar, zeigen keine Sedementationstendenz und haben eine aktivierte Oberfläche zum Koppeln von Biomolekülen oder chemisch reagierenden Indikatoren. Im Falle der Verwendung des Ruthenium-(II)-tris(4,7-diphenyl-1,10-phenanthrolin)-Komplexes als phosphoreszierendem Farbstoff erhält man helle lumineszierende Nanopartikel mit starken Stokes-Verschiebungen. In wässrigen Lösungen war kein Auswaschen von Farbstoffen zu beobachten. Sie können entweder mit einem blauen Argonionenlaser oder mit hellen Blaulicht-emittierenden Dioden (LED's) erregt werden.

Der Ausfällungsprozess gestattet das gleichzeitige Einbetten unterschiedlicher phosphoreszierender und fluoreszierender Farbstoffe in einem individuellen Nanopartikel.

Der langlebige phosphoreszente Lumineszenzdonor RU(dph-phen)$_3$(TMS)$_2$ zeigt eine große Stokes-Verschiebung von etwa 150 nm ($\lambda_x$ = 467 nm, $\lambda_m$ = 613 nm) eine hohe Quantenausbeute ($\phi$ > 40 %), einen großen Extinktionskoeffizienten ($\in$ = 28,100 LMol$^{-1}$ x cm$^{-1}$), und ist lipophil, um ein Ausdünnen in wässriger Umgebung zu vermeiden. Er kann mit einem Argon- Ionenlaser bei $\lambda_x$ = 488 nm erregt werden. Schließlich ist sein Emissionsspektrum breit genug, um mit den Absorptionsspektren verschiedener lumineszierender Akzeptorfarbstoffe zu überlappen. Während des Herstellungsprozesses werden die vollständig in den Partikel eingebaut.

Fluoreszente Carbocyanine wirken als Lumineszenz-Energieakzeptoren. Der Vorteil dieser Indikatorfarbstoffe ist, dass keine Selbstabsorption bei der Erregungswellenlänge des Rutheniumkomplexes von 488 nm zeigen. Wegen ihrer hohen Extinktionskoeffizienten $\in$ von mehr als 200.000 L Mol$^{-1}$ cm$^{-1}$, ihrem lipophilen Charakter, ihren großen Überlappungsintegralen mit dem Rutheniumdonorfarbstoff und schließlich ihre leichte Erhältlichkeit im Handel, machen Carbocyaninfarbstoffe zu idealen Energieakzeptoren.

Tabelle 7 fasst die spektralen Daten der hier verwendeten Donor- und Akzeptorfarbstoffe in DMF zusammen.

TABELLE 7

| Spekrale Charakterisierung der Ruthenium Donor und Carbocyanin Akzeptor Farbstoffe. | | | | | |
|---|---|---|---|---|---|
| Farbstoff | Lösungsmittel | $\lambda_{max}$ (nm) | $\lambda_{em}$ (nm) | $\Delta\lambda$ (nm) | $\varepsilon$ (L mol$^{-1}$ cm$^{-1}$) |
| RU[a] | Phosphatpuffer | 465 | 612 | 147 | 28,100 |
| CY582 | DMF | 587 | 608 | 21 | 224,700 |
| CY604 | DMF | 612 | 633 | 21 | 238,300 |
| CY655 | DMF | 659 | 678 | 19 | 245,400 |
| CY703 | DMF | 713 | 731 | 18 | 324,500 |

[a] eingeschlossen in Nanopartikeln (= Lösung C1a)

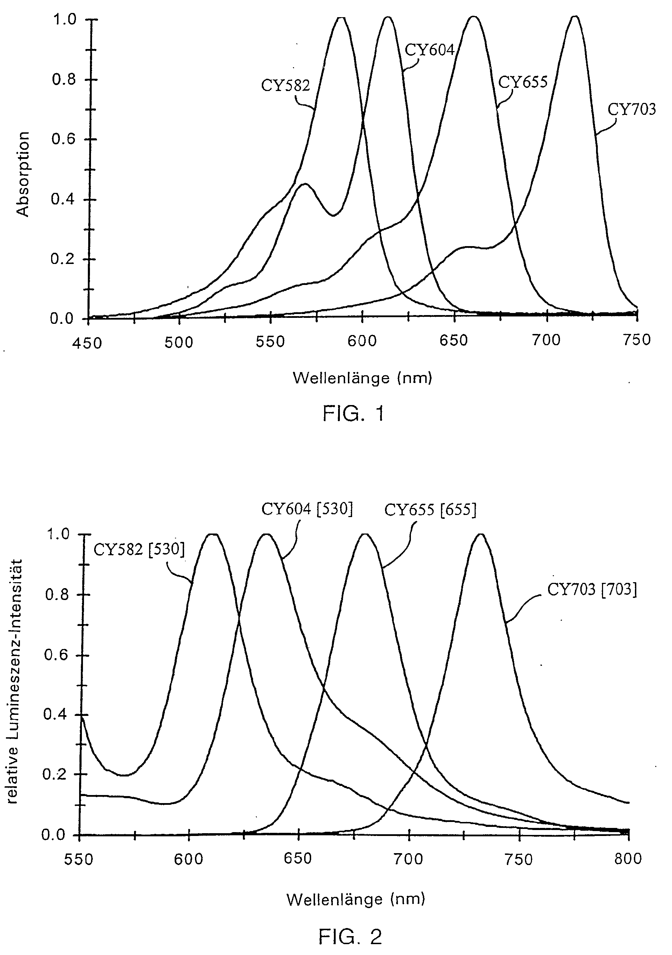

Die Figuren 1 und 2 zeigen normalisierte Absorptions- und Fluoreszenz-Emissionsspektren der benutzten Carbocyaninfarbstoffe in DMF.

Man erhält eine zweidimensionale Anordnung von Multiplex-Markern, wobei die erste Dimension die Absorptionswellenlänge λ der Carbocyanin-Akzeptorfarbstoffe ist und die zweite Dimension die Lumineszenz-Abklingzeit $T$.

Es können sogar sieben oder acht unterschiedliche Carbocyaninfarbstoffe als Lumineszenzenergie-Akzeptoren verwendet werden, solange ihre Erregungswellenlängen die Ruthenium-Donoremissionswellenlänge im Bereich von angenähert 590 nm bis 750 nm abdecken. Durch spektrale Überlappung eines Farbstoffpaares wird Energietransfer möglich, und Phosphoreszenz wird auf Fluoreszenzindikatoren übertragen, wie etwa langwellig erregbare Carbocyaninfarbstoffe. Hierdurch lassen sich phosphoreszierende Nanopartikel mit einer außerordentlich großen Stokes-Verschiebung bis zu 300 nm herstellen. Diese Nanopartikel können als helle phosphoreszierende Marker bei der Immuno- oder DNA-Sensierung oder als Nanosonden zum Messen intrazellulärer chemischer Parameter benutzt werden. Ferner bilden sie ausgezeichnete Phosphoreszenzstandards und sind bei der Ausgestaltung phosphoreszierender chemischer Sensoren nützlich.

5. Charakterisierung der Nanopartikel

Tabelle 8 zeigt eine Zusammenfassung der spektralen Charakterisierung vier verschiedener Carbocyanin-Nanopartikel mit verschiedenen Farbstoffkonzentrationen in Phosphatpufferlösung (pH 7,0; IS = 20 mmol).

TABELLE 8

| Nanopartikel-Charakterisierung der Ruthenium Donor - Carbocyanin Akzeptor Paare in Phosphatpufferlösung | | | | | | |
|---|---|---|---|---|---|---|
| Lösung | Carbocyanin-Akzeptor | c (Akzeptor)[a] (μmol/L) | $\tau$, Luft (μs) | $\phi$, Luft | $\phi$, $Na_2SO_3$ | $\Delta\phi$ (%) |
| C1a | CY582 | 0 | 6.23 | 0.37 | 0.39 | -5.1 |
| C1b | CY582 | 4.11 | 5.14 | 0.36 | 0.37 | -2.7 |
| C1c | CY582 | 8.22 | 4.58 | 0.34 | 0.35 | -2.9 |
| C1d | CY582 | 20.56 | 2.59 | 0.32 | 0.33 | -3.0 |
| C1e | CY582 | 41.12 | 1.03 | 0.27 | 0.28 | -3.6 |
| C2b | CY604 | 5.14 | 2.87 | 0.32 | 0.32 | >-1.0 |
| C2c | CY604 | 10.28 | 1.77 | 0.25 | 0.25 | >-1.0 |
| C2d | CY604 | 25.71 | 0.63 | 0.08 | 0.08 | >-1.0 |
| C2e | CY604 | 51.42 | 0.39 | 0.03 | 0.03 | >-1.0 |
| C3a | CY655 | 0 | 6.00 | 0.32 | 0.33 | -3.0 |
| C3b | CY655 | 3.86 | 4.15 | 0.30 | 0.31 | -3.2 |
| C3c | CY655 | 7.71 | 1.78 | 0.27 | 0.28 | -3.6 |
| C3d | CY655 | 19.29 | 0.85 | 0.18 | 0.19 | -5.3 |
| C3e | CY655 | 38.57 | 0.38 | 0.07 | 0.08 | -12.5 |
| C4b | CY703 | 11.10 | 3.97 | 0.25 | 0.26 | -3.8 |
| C4c | CY703 | 22.20 | 2.56 | 0.20 | 0.21 | -4.8 |
| C4d | CY703 | 55.51 | 1.46 | 0.16 | 0.16 | >-1.0 |
| C4e | CY703 | 111.02 | 1.16 | 0.06 | 0.06 | >-1.0 |

[a] c (RU-Donor) = 10.00 μmol/L (konstant)

Hierbei bedeutet c in der dritten Spalte die Konzentration des Akzeptors, $T$ in der vierten Spalte die Abklingzeit, und $\phi$ in den fünften bis siebten Spalten die Quantenausbeute.

Das resultierende zweidimensionale Feld von Multiplex-Markern zeigt ähnliche Merkmale bei der Erregung mit einem Argonionenlaser bei 488 nm. Die durchschnittliche Abklingzeit nimmt zu, und zwar in Abhängigkeit vom verwendeten Carbocyanin und dessen Konzentration.

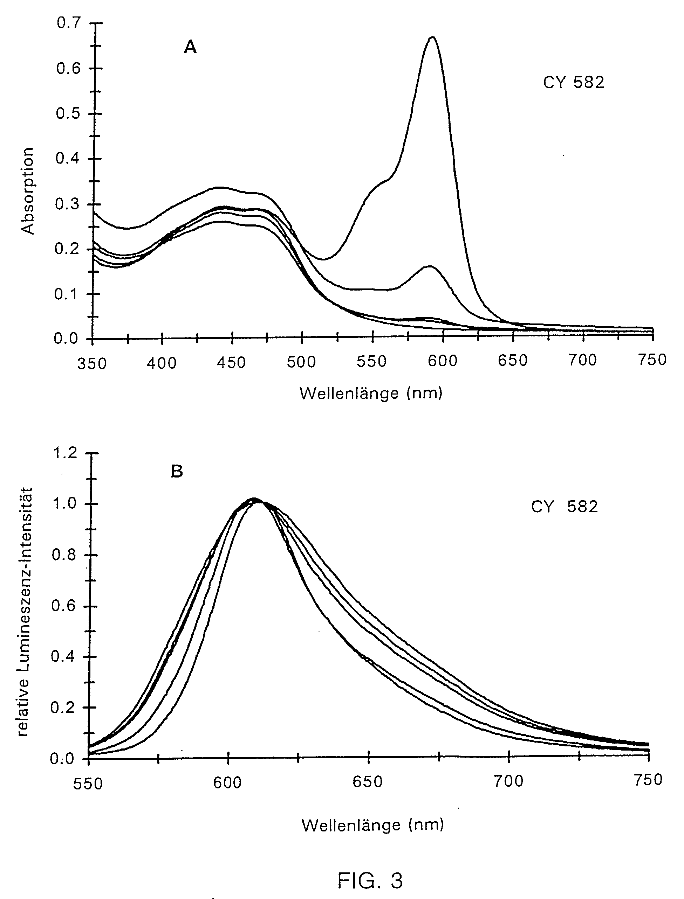

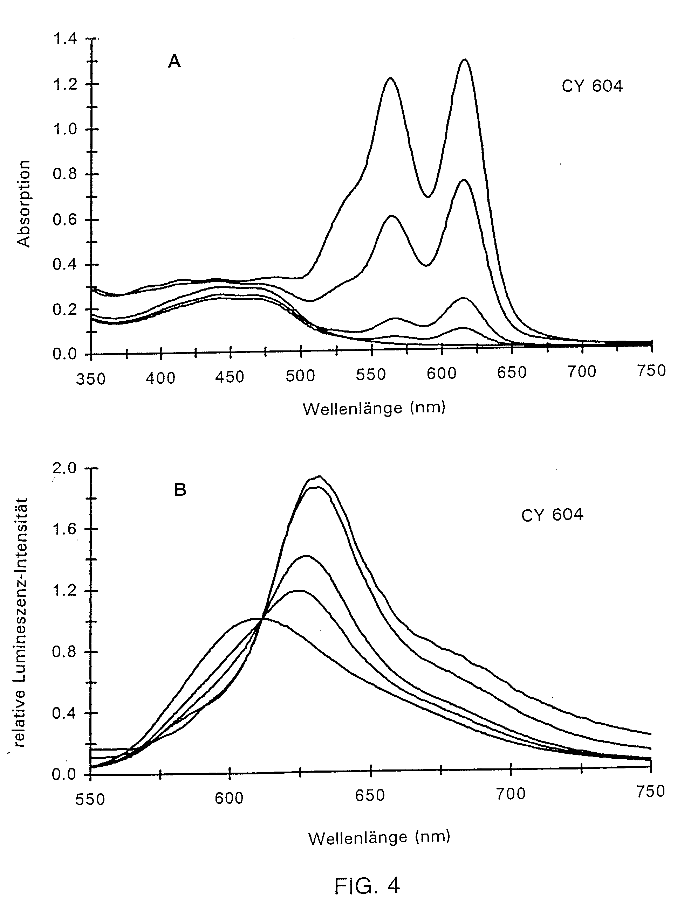

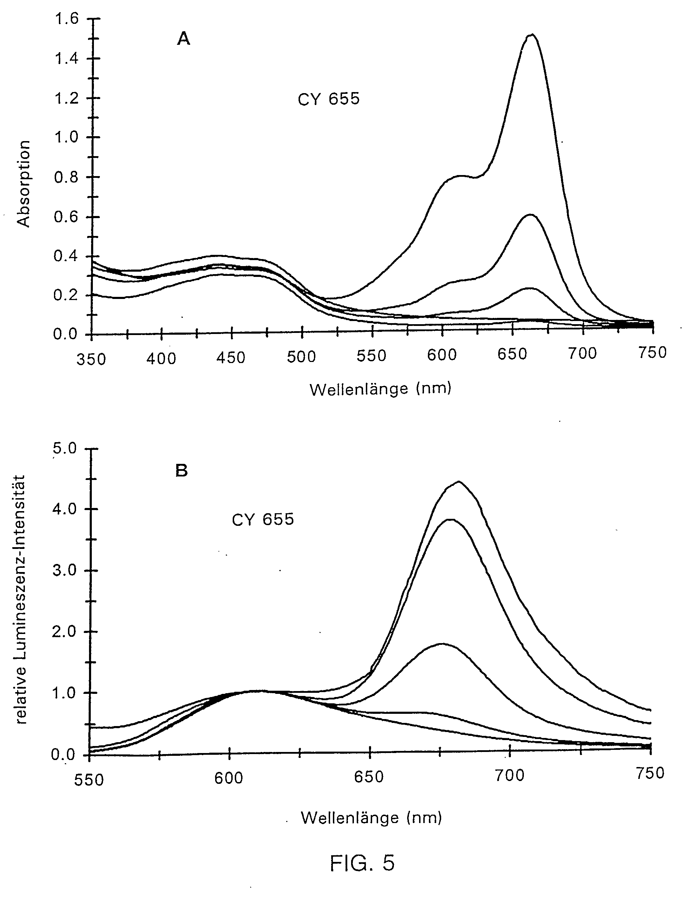

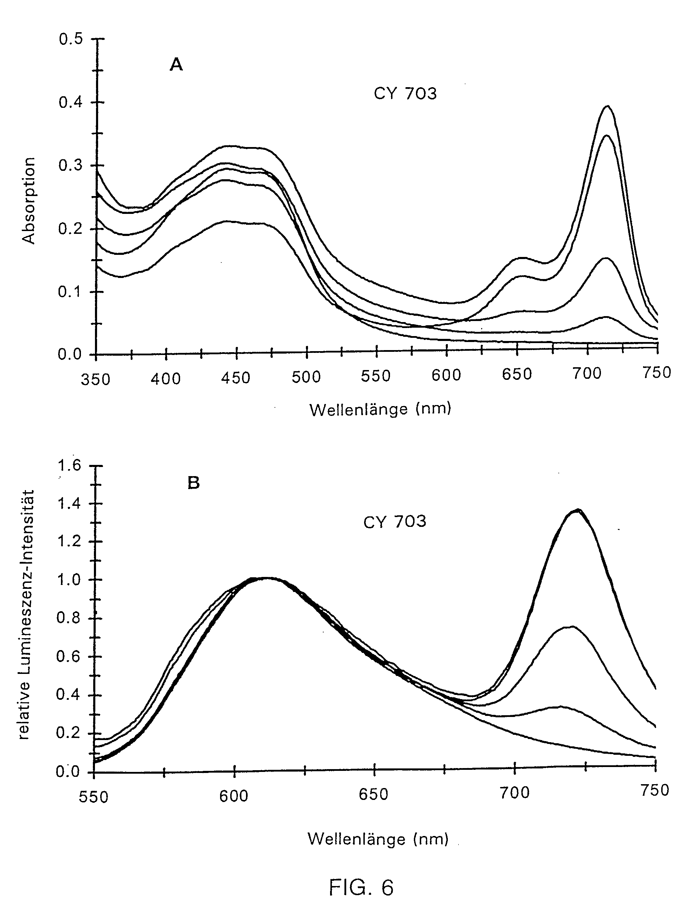

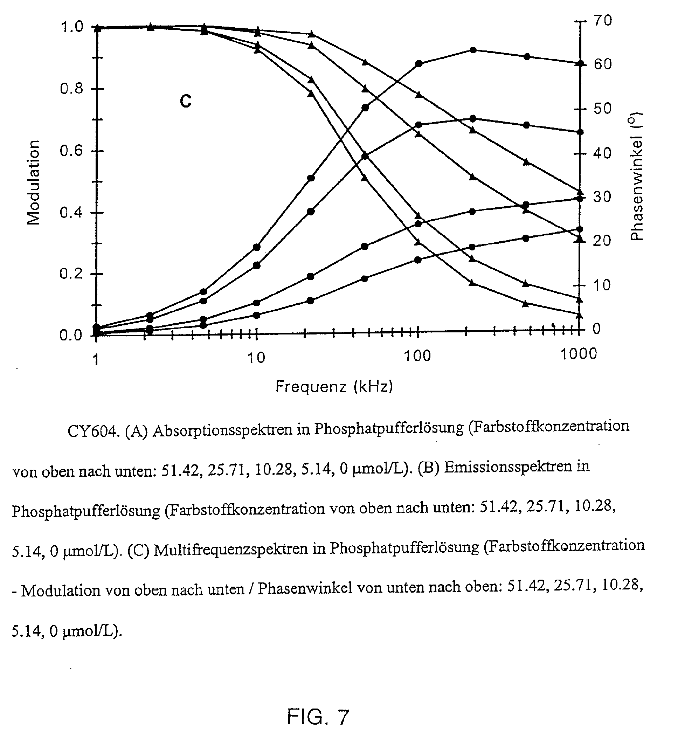

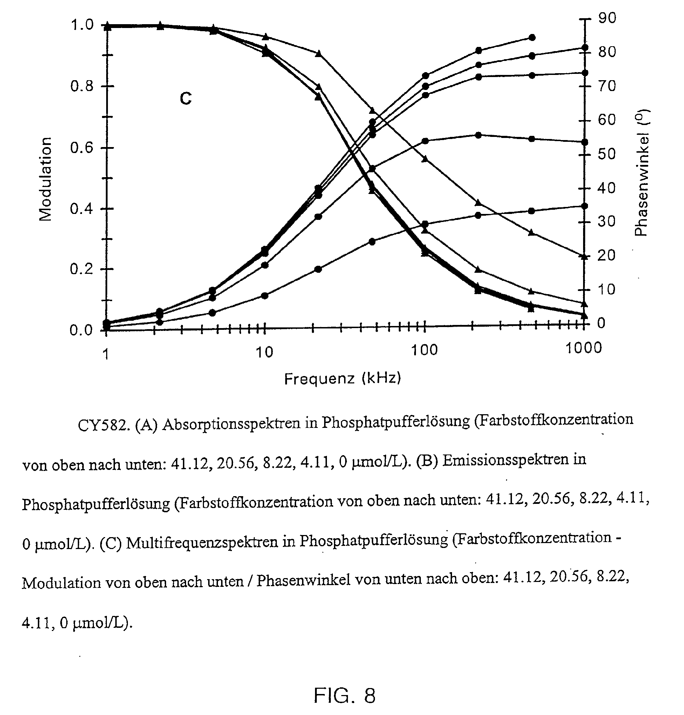

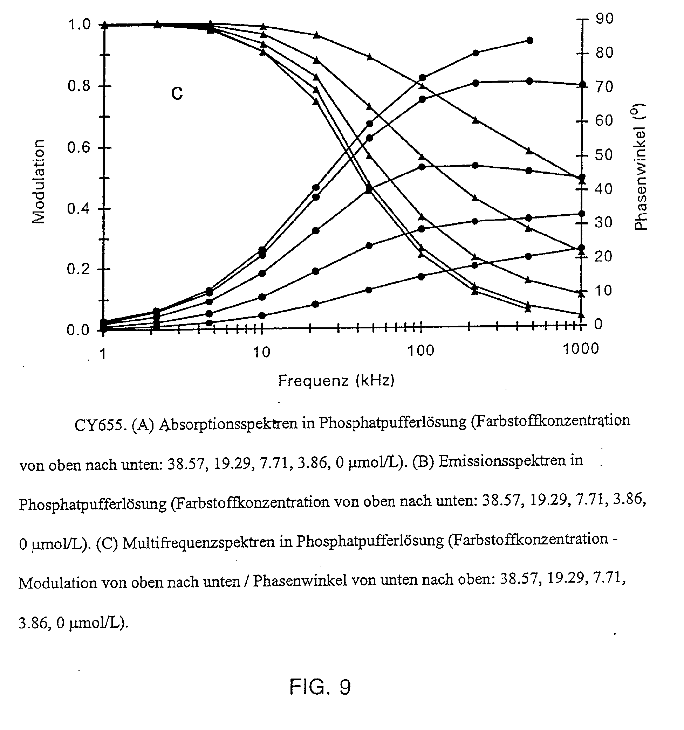

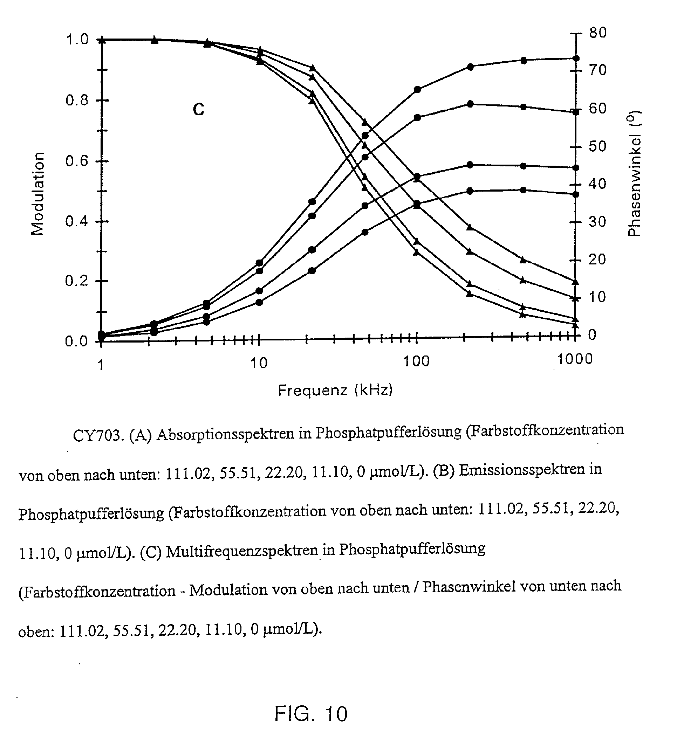

Die Figuren 3 bis 6 zeigen jeweils oben (A) die Absorptionsspektren der Nanopartikel jeweils verschiedener Carbocyanintypen (CY582, CY604, CY655 und CY703) mit jeweils verschiedenen Konzentrationen in Phosphatpufferlösung, und jeweils unten (B) die Emissionsspektren ($\lambda_x$ = 488 nm) der jeweiligen Partikel, die bei der Emissionswellenlänge des Rutheniumdonorkomplexes (611,5 nm) auf 1 normalisiert sind. Die Figuren 7 bis 10 zeigen jeweils den Phasenwinkel und die Modulation in einem Frequenzbereich von 1 kHz bis 1 MHz der Partikel der Figuren 3 bis 6.

Die Fluoreszenz-Emission des Rutheniumdonorkomplexes nimmt aufgrund des Energietransfers zu dem Carbocyaninakzeptor in ein und demselben Nanopartikel ab. Ferner wurden die fotophysikalischen Eigenschaften, nämlich die Tendenz der Nanopartikel zur Aggregation und deren Stabilität geprüft. In Phosphatpufferlösung bei pH 7,00 mit einer Ionenstärke (eingestellt mit NaCl von 20 mmol), waren die Partikel über mehrere Wochen hinweg stabil. Die Suspensionen sollten lichtgeschützt und bei etwa 10 °C gekühlt aufbewahrt werden.

Zusätzlich zu den spektralen Charakterisierungen der Partikel wurden deren physikalische Eigenschaften geprüft. Rasterelektronenmikroskopische Bilder der Partikel zeigten eine nahezu kreisförmige Form und einen Durchmesser von ca. 50 nm. Die statische und dynamische Lichstreuung bei Laser-Doppleranämometrie-Experimenten ergaben einen polydispersen Coil mit einem Partikeldurchmesser von 100 bis 50 nm und einem Zeta-Potential, welches die negative Oberflächenladung infolge der Carboxylgruppen bestätigte, wie in Tabelle 9 gezeigt.

Tabelle 9

| Partikelgröße und Oberflächenladung der Lösung C3a bei dynamischen Lichtstreuungsexperimenten | | | |
|---|---|---|---|
| c(Ru(dph-phen)$_3$(TMS)$_2$) [µmol] 39,6 | c(CY655) [µmol/l] 0,0 | hydrodynamischer Durchmesser [nm] 84,7 | ζ-Potenzial [mV] -58,0 ± 0,7 |

## Patentansprüche

1.  Anordnung zur fluorometrischen Messung eines Analyten, umfassend:

    einen lumineszierenden Donorfarbstoff und mehrere, gemeinsam mit dem Donorfarbstoff immobilisierte, durch Energietransfer von dem Donorfarbstoff lumineszierende Akzeptorfarbstoffe,

    **dadurch gekennzeichnet,**
    **dass** der Donorfarbstoff ein Phosphoreszenzfarbstoff ist und die jeweiligen Akzeptorfarbstoffe Fluoreszenzfarbstoffe sind.

2.  Anordnung nach Anspruch 1,
    **dadurch gekennzeichnet,**
    **dass** nur ein einziger Donorfarbstoff immobilisiert ist.

3.  Anordnung nach Anspruch 1 oder 2,
    **dadurch gekennzeichnet,**
    **dass** der Donorfarbstoff auf blaues Licht anspricht und bevorzugt ein Ruthenium-(II)-polypyridkomplex ist.

4.  Anordnung nach einem der vorhergehenden Ansprüche,
    **dadurch gekennzeichnet,**
    **dass** die mehreren Akzeptorfarbstoffe unterschiedliche Emissionsspektren aufweisen oder/und in Wechselwirkung mit dem Donorfarbstoff unterschiedliche Lumineszenz-Abklingzeiten der Anordnung hervorrufen.

5.  Anordnung nach einem der vorhergehenden Ansprüche,
    **dadurch gekennzeichnet,**
    **dass** ein Akzeptorfarbstoff jeweils separat in mehreren unterschiedlichen Konzentrationen mit dem Donorfarbstoff immobilisiert vorliegt.

6.  Anordnung nach einem der vorhergehenden Ansprüche,
    **dadurch gekennzeichnet,**
    **dass** die Akzeptorfarbstoffe jeweils Carbocyaninfarbstoffe sind.

7.  Anordnung nach einem der vorhergehenden Ansprüche,
    **dadurch gekennzeichnet,**
    **dass** in eine den Donorfarbstoff und die Akzeptorfarbstoffe immobilisierende Kunststoffmatrix der Donorfarbstoff mit einer Konzentration von 1 bis 15 Gew.-%, bevorzugt ca. 10 Gew.-% eingebettet ist.

8.  Anordnung nach einem der vorhergehenden Ansprüche,
    **dadurch gekennzeichnet,**
    **dass** der Donorfarbstoff und die Akzeptorfarbstoffe in Mikro- oder Nanopartikel eingebettet sind, bevorzugt in einer Größe im Bereich von 50 µm.

9.  Anordnung nach Anspruch 8,
    **dadurch gekennzeichnet,**

**dass** die Mikro- oder Nanopartikel hergestellt sind durch Fällung einer Lösung von Polynitril in Dimethylformamid (DMF).

10. Anordnung nach einem der Ansprüche 1 bis 9,
**dadurch gekennzeichnet,**
**dass** der Donorfarbstoff eine Lumineszenzabklingzeit im Bereich von 100 ns bis 100 µs, vorzugsweise 100 ns bis 10 µs, aufweist.

11. Anordnung nach einem der Ansprüche 1 bis 10,
**dadurch gekennzeichnet,**
**dass** die Akzeptorfarbstoffe hinsichtlich der durch den Donorfarbstoff stimulierten Lumineszenz eine Lumineszenz-Abklingzeit von ≥50 ns, vorzugsweise 50 ns bis 10 µs, aufweisen.

12. Verfahren zur gleichzeitigen fluorometrischen Messung mehrerer Analyten, insbesondere mittels einer Anordnung nach einem der vorhergehenden Ansprüche, die einen phosphoreszierenden Donorfarbstoff und mehrere unterschiedliche, hiermit immobilisierte fluoreszierende Akzeptorfarbstoffe aufweist, mit den Schritten:

- Anregen des Donorfarbstoffs,
- Messen und Auswerten der spektral unterschiedlichen Fluoreszenzantworten der Akzeptorfarbstoffe und
- Messen und Auswerten der durch die Fluoreszenzsignale der Akzeptorfarbstoffe in Wechselwirkung mit dem Donorfarbstoff beeinflussten Lumineszenzabklingzeiten.

13. Verfahren nach Anspruch 12,
**dadurch gekennzeichnet,**
**dass** eine zeitlich aufgelöste Messung und Auswertung der Fluoreszenzantworten der Akzeptorfarbstoffe oder/und der Lumineszenzabklingzeiten zur Reduzierung des Untergrundsignals erfolgt.

FIG. 1

FIG. 2

FIG. 3

FIG. 4

FIG. 5

FIG. 6

CY604. (A) Absorptionsspektren in Phosphatpufferlösung (Farbstoffkonzentration von oben nach unten: 51.42, 25.71, 10.28, 5.14, 0 μmol/L). (B) Emissionsspektren in Phosphatpufferlösung (Farbstoffkonzentration von oben nach unten: 51.42, 25.71, 10.28, 5.14, 0 μmol/L). (C) Multifrequenzspektren in Phosphatpufferlösung (Farbstoffkonzentration - Modulation von oben nach unten / Phasenwinkel von unten nach oben: 51.42, 25.71, 10.28, 5.14, 0 μmol/L).

FIG. 7

CY582. (A) Absorptionsspektren in Phosphatpufferlösung (Farbstoffkonzentration von oben nach unten: 41.12, 20.56, 8.22, 4.11, 0 µmol/L). (B) Emissionsspektren in Phosphatpufferlösung (Farbstoffkonzentration von oben nach unten: 41.12, 20.56, 8.22, 4.11, 0 µmol/L). (C) Multifrequenzspektren in Phosphatpufferlösung (Farbstoffkonzentration - Modulation von oben nach unten / Phasenwinkel von unten nach oben: 41.12, 20.56, 8.22, 4.11, 0 µmol/L).

FIG. 8

CY655. (A) Absorptionsspektren in Phosphatpufferlösung (Farbstoffkonzentration von oben nach unten: 38.57, 19.29, 7.71, 3.86, 0 μmol/L). (B) Emissionsspektren in Phosphatpufferlösung (Farbstoffkonzentration von oben nach unten: 38.57, 19.29, 7.71, 3.86, 0 μmol/L). (C) Multifrequenzspektren in Phosphatpufferlösung (Farbstoffkonzentration - Modulation von oben nach unten / Phasenwinkel von unten nach oben: 38.57, 19.29, 7.71, 3.86, 0 μmol/L).

FIG. 9

CY703. (A) Absorptionsspektren in Phosphatpufferlösung (Farbstoffkonzentration von oben nach unten: 111.02, 55.51, 22.20, 11.10, 0 μmol/L). (B) Emissionsspektren in Phosphatpufferlösung (Farbstoffkonzentration von oben nach unten: 111.02, 55.51, 22.20, 11.10, 0 μmol/L). (C) Multifrequenzspektren in Phosphatpufferlösung (Farbstoffkonzentration - Modulation von oben nach unten / Phasenwinkel von unten nach oben: 111.02, 55.51, 22.20, 11.10, 0 μmol/L).

FIG. 10